# FASCICULE DE BREVET EUROPEEN

(11) **EP 3 024 566 B1**
(45) Date de publication et mention de la délivrance du brevet: **01.05.2019**
(21) Numéro de dépôt: 14750579.6
(22) Date de dépôt: 24.07.2014
(51) Int. Cl.: B01J 13/02, A61K 9/51

(54) **NANOPARTICULES LIPIDIQUES MULTICOMPARTIMENTÉES**
LIPIDNANOPARTIKEL MIT MEHREREN SEGMENTEN
MULTICOMPARTMENTAL LIPID NANOPARTICLES

(30) Priorité: 25.07.2013 FR 1357363
(43) Date de publication de la demande: 01.06.2016
(73) Titulaire: Centre National de la Recherche Scientifique (C.N.R.S.), 75794 Paris Cedex 16 (FR); Universite Paris-Sud, 91400 Orsay (FR)
(72) Inventeur: FAIVRE, Vincent, F-75003 Paris (FR); LESIEUR, Sylviane, F-92330 Sceaux (FR); OLLIVON, Michel, F-94120 Fontenay sous Bois (FR); OUATTARA, Modibo, BP 506 Korhogo (CI); TRUONG CONG, Tri, Ho Chi Minh 30 (VN)
(74) Mandataire: Lavoix
(86) Numéro de dépôt international: PCT/FR2014/051922
(87) Numéro de publication internationale: WO 2015/011419

(56) Documents cités:
- US-A- 5 662 932
- H Heiati ET AL: "Evidence for phospholipid bilayer formation in solid lipid nanoparticles formulated with phospholipid and triglyceride", Pharmaceutical research, 1 septembre 1996 (1996-09-01), pages 1406-1410, XP055108356, UNITED STATES Extrait de l'Internet: URL:http://www.ncbi.nlm.nih.gov/pubmed/889 3283 [extrait le 2014-03-17]
- BÉATRICE HEURTAULT ET AL: "A Novel Phase Inversion-Based Process for the Preparation of Lipid Nanocarriers", PHARMACEUTICAL RESEARCH, SPRINGER NEW YORK LLC, US, vol. 19, no. 6, 1 juin 2002 (2002-06-01), pages 875-880, XP002539234, ISSN: 0724-8741, DOI: 10.1023/A:1016121319668

## Description

### Domaine technique

La présente invention se place dans le cadre du développement de nanotechnologies pour l'administration de principes actifs.

La présente invention se rapporte à des nanoparticules lipidiques multicompartimentées, (ci-après dénommées cellisomes), leur procédé de préparation et leur utilisation comme vecteur pour l'administration de molécule d'intérêt, notamment par voie injectable, orale nasale ou cutanée.

Dans la description ci-dessous, les références entre crochets (**[]**) renvoient à la liste des références présentée à la fin du texte.

### Etat de la technique

Durant les trente dernières années, deux grandes catégories de systèmes nanométriques de vecteurs de molécules d'intérêt ont été développées : les systèmes polymériques et lipidiques.

Les premiers d'entre eux se sont montrés relativement décevant du point de vue de leur industrialisation, probablement pour des raisons de toxicité. Aussi les formulations disponibles sur le marché sont majoritairement à base de lipides qui ont généré deux grandes familles de vecteurs : les liposomes et les particules lipidiques (nanoémulsion (NE), les nanoparticules lipidiques nanostructurées (NLC), les nanoparticules lipidiques solides (SLN)). Les liposomes, et les nanoémulsions dans une moindre mesure, ont donné lieu à de nombreuses applications cosmétiques et plusieurs médicaments sur le marché, alors que les particules lipidiques nanostructurées ou solides, de mise au point plus récente, sont présentes dans de nombreux produits cosmétiques et en essais cliniques pour le domaine pharmaceutique.

Les liposomes sont définis comme une structure artificielle constituée d'une ou plusieurs bicouches lipidiques concentriques, emprisonnant entre elles des compartiments d'eau ou de tampon aqueux. Les liposomes sont préparés à partir d'un seul ou plusieurs types de phospholipides naturels ou de synthèse qui s'organisent de sorte que les têtes polaires se regroupent entre elles pour permettre l'établissement de la bicouche. La façon la plus classique de préparer des liposomes est celle dite de l'hydratation d'un film lipidique. Les liposomes sont de plus en plus développés en tant que vecteurs de principes actifs qu'ils soient hydrosolubles, liposolubles ou amphiphiles. Leur encapsulation dans la phase aqueuse ou dans la bicouche lipidique permet ainsi de protéger les principes actifs vis-à-vis d'une dégradation enzymatique ou d'une élimination par le système immunitaire, mais également de diminuer leurs éventuels effets secondaires toxiques (*e.g.* hémolyse, thrombophlébite, coagulation sanguine) lors d'une administration parentérale. (Meure et al., Aaps Pharmscitech, 9: 798-809, 2008 ; Storm et Crommelin, Pharmaceutical Science & Technology Today, 1 : 19-31, 1998) [1, 2]. A la base d'une douzaine de compositions commerciales (*e.g.* Myocet®, Doxil®/Caelyx®, AmBisome®, Visudyne®, etc...), les liposomes présentent cependant plusieurs inconvénients majeurs : un manque de spécificité pour la cellule cible, une oxydation et instabilité physique des phospholipides nécessitant leur lyophilisation, une production à l'échelle industrielle délicate, et une certaine limitation dans la quantité de molécules d'intérêt encapsulables. En effet, des molécules amphiphiles ou lipophiles sont capables de s'associer aux liposomes par insertion dans leurs membranes mais au risque d'une déstabilisation de cette dernière.

Les émulsions sont de fines dispersions de gouttelettes d'un liquide (phase dispersée) dans un autre (phase dispersante ou continue) qui lui est très peu miscible ; elles sont le plus souvent de type huile/eau. On parle de nanoémulsions (NE) lorsque la granulométrie obtenue est très fine, c'est-à-dire une taille moyenne de l'ordre de la centaine de nanomètres. Elles sont généralement fabriquées par fragmentation mécanique d'une phase huileuse dans une phase aqueuse, et éventuellement stabilisées par la présence de tensioactif. Par rapport à des émulsions classiques, la petite taille des globules leur confère des propriétés intéressantes sur le plan pharmaceutique, notamment en termes de stabilité physique pendant le temps de stockage et de voie d'administration possible, en particulier la voie intra-veineuse qui requiert l'utilisation de gouttelettes de petites tailles. Toutefois, ces systèmes ne peuvent incorporer que des principes actifs très lipophiles solubles dans les huiles constitutives de ces émulsions (huile de soja, d'olives), limitant ainsi leurs applications potentielles.

Les nanoparticules lipidiques solides (SLN) et lipidiques nanostructurées (NLC) ont été développées afin d'augmenter la stabilité physicochimique des principes actifs encapsulés et la stabilité après administration des vecteurs lipidiques dans leur ensemble, pour généralement les exploiter à terme en cosmétologie du fait de leurs propriétés d'adhésivité, d'occlusion et d'hydratation de la peau et en pharmacie pour l'administration et la protection de principes actifs d'intérêt. (Bunjes, Current Opinion in Colloid & Interface Science, 16(5) : 405-411, 2011 ; Harde et al., Expert Opinion on Drug Delivery, 8(11) : 1407-1424, 2011 ; Harms et al., Journal of Drug Delivery Science and Technology, 21(1) : 89-99, 2011 ; Joshi et Muller, European Journal of Pharmaceutics and Biopharmaceutics, 71 : 161-172, 2009; Muller et al., Current Drug Discovery technologies, 8(3) : 207-227, 2011 ; Pardeike et al., International Journal of Pharmaceutics, 366: 170-184, 2009; Souto et Doktorovova, Methods in Enzymology, 464 : 105-129, 2009) [3-9]. Comme dans le cas des nanoémulsions décrites précédemment, la très grande lipophilie des matières premières utilisées limite le choix des principes actifs potentiellement administrables. De plus, il a été montré que le polymorphisme des lipides à l'état solide influe grandement sur la stabilité physique de ces systèmes (expulsion des molécules actives, gélification), notamment dans le cas des SLN.

Des nanocapsules lipidiques développées par l'Université d'Angers ont été obtenues par un procédé d'inversion de phase et sont entourées par une monocouche de phospholipides. Bien que très proches des nanodispersions de type SLN et NLC, ces particules ont été décrites comme des nanocapsules stabilisées par une couche cristallisée de phospholipides et d'un tensioactif non-ionique polyoxyéthyléné (Demande Internationale WO 01/64328 ; Huynh et al., Journal of Pharmaceutics, 379 : 201-209, 2009) [10, 11]. Intéressante car relativement « douce », la technique d'inversion de phase nécessite l'utilisation de matières premières relativement spécifiques et un contrôle très fin des températures pendant le procédé de préparation. Ces aspects peuvent présenter une limite au développement de cette approche à très grande échelle.

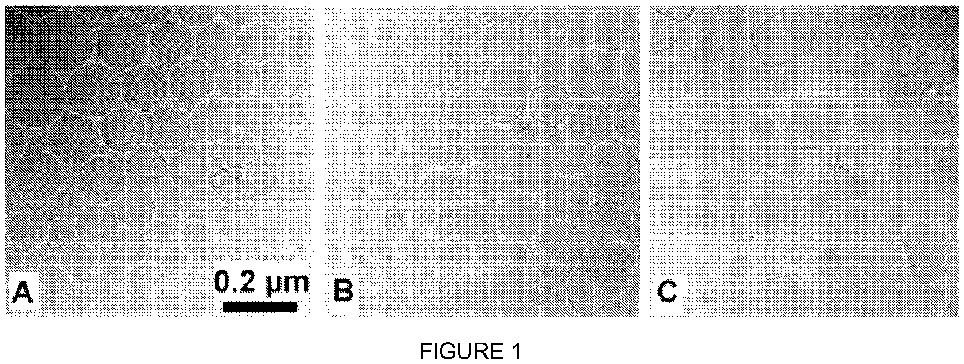

Il y a quelques années des émulsions cationiques submicroniques composées de structures bicompartimentales huile/eau, nommées « sacs à main » ou « hand-bags » ont été développées (figure 1). Leur formation nécessite la présence de stéarylamine qui favoriserait l'insertion massive de triglycérides dans la bicouche lipidique délimitant le compartiment aqueux, pour les exploiter à terme comme vecteur de principes actifs (Texeira et al., Pharmaceutical Research, 17: 1329-1332, 2000) [12]. Malheureusement, la proportion de ces objets bicompartimentés reste minoritaire (moins de 20%) parmi une multitude d'autres objets formés pendant la préparation (micelles, liposomes, nanoémulsions). De plus, l'utilisation d'un tensioactif cationique comme la stéarylamine est à envisager avec précaution compte tenu de la toxicité de ce type de produit vis-à-vis des membranes biologiques chargées négativement.

Plus marginales, des nanoémulsions de particules ayant un diamètre de 10-250 nm (nommées émulsomes ou ultrasomes) comprenant un coeur lipidique composé d'un lipide sous forme liquide ou solide entouré et stabilisé par au moins une bicouche phospholipidique, comme dans les liposomes, ont été développées et destinées à l'administration par voie parentérale, orale, rectale, intranasale ou topique de molécules liposolubles ou hydrosolubles (Brevet US 5,576,016 ; Gupta et al., Journal of Drug Targeting, 15 : 437-444, 2007; Gupta et Vyas, Journal of Drug Targeting, 15 : 206-217, 2007 ; Kretschmar et al., Mycoses, 44 : 281-286, 2001 ; Paliwal et al., International Journal of Pharmaceutics, 380 : 181-188, 2009 ; Wu et al., Journal of Immunology, 185(6) : 3401-3407, 2010) [13-18]. Le procédé de préparation de ces émulsomes est difficilement exploitable à l'échelle industrielle car il nécessite en général l'utilisation d'un solvant organique, le dépôt et la réhydratation d'un film phospholipidique. En effet, ces particules sont principalement obtenues par une technique d'hydratation d'un film phospholipidique très proche de celle employée pour les liposomes exceptée que la phase aqueuse contient des nanoparticules lipidiques préformées. La particule finale résulte de « l'emprisonnement statistique » des gouttelettes huileuses dans des bicouches phospholipidiques. En conséquence, le procédé génère *a priori* différentes populations d'objets (émulsomes, liposomes, nanoémulsions ou nanoparticules solides) sans solidarisation entre les parties lipidiques et phospholipides. Ceci peut *a priori* induire des problèmes de stabilité des systèmes au cours de certaines opérations de purification (centrifugation par exemple) ou pendant le stockage.

US 5 662 932 A décrit des particules lipidiques ayant un diamètre de 20 à 180 nm, caractérisées en ce qu'elles comprennent un coeur lipidique enrobé par un compartiment aqueux délimité par une bicouche phospholipidique dans un porteur pharmaceutiquement acceptable.

Il existe donc un réel besoin de vecteurs lipidiques palliant ces défauts, inconvénients et obstacles de l'art antérieur, en particulier d'un procédé de production simple permettant de maîtriser la stabilité des vecteurs lipidiques sur le long terme pour l'administration de large quantité de molécules d'intérêt ayant une large gamme de polarité, et éventuellement d'envisager la co-encapsulation de principes actifs hydrophiles et lipophiles dans un même nano-objet

### Description de l'invention

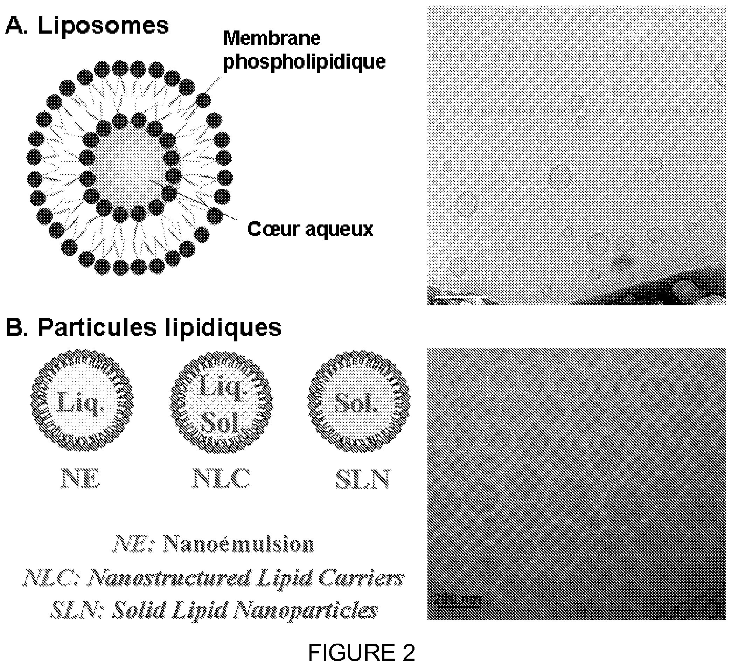

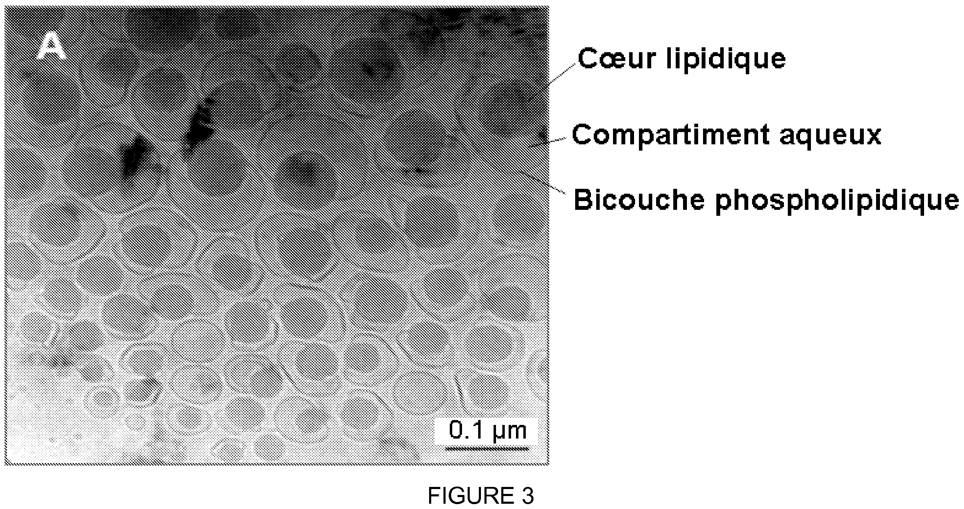

S'appuyant sur leurs propres expériences autour de la stabilité et la toxicité des nanosystèmes lipidiques, les Inventeurs ont développé de nouvelles nanoparticules lipidiques multicompartimentées (ou cellisomes) représentant un système hybride entre ceux précédemment décrits, à savoir les liposomes et les particules lipidiques (figure 2A-B) dans lequel une matrice lipidique et un compartiment aqueux sont associés au sein d'un même objet de dimension nanométrique. L'originalité principale des cellisomes de l'invention provient de leur morphologie multicompartimentée (figure 3) malgré un procédé de préparation simple (Homogénéisation Haute Pression) et transposable à l'échelle industrielle. Par ailleurs les matières premières utilisées sont peu onéreuses, déjà reconnues par diverses pharmacopées et employées dans l'industrie pharmaceutique.

D'un point de vue morphologique, ces nanoparticules multicompartimentées de l'invention sont totalement différentes des émulsomes. Dans ces derniers, l'objet est isotrope (depuis le centre, mêmes propriétés dans toutes les directions) car le compartiment lipidique est englobé dans une ou plusieurs bicouches phospholipidiques, elles-mêmes délimitant des compartiments aqueux concentriques. Dans le cas des nanoparticules lipidiques multicompartimentées (ou cellisomes) de l'invention, l'organisation est anisotrope (depuis le centre, propriétés différentes selon la direction) car le compartiment lipidique n'est que partiellement enrobé par le compartiment aqueux.

Dans les cellisomes de l'invention, le coeur lipidique peut être constitué de mélanges lipidiques liquides ou semi-solides à température ambiante (25°C) incorporant des glycérides et des esters de polyéthylèneglycol. Ce dernier aspect est très important pour permettre l'encapsulation dans lesdites nanoparticules de principes actifs (PA) ayant une large gamme de polarité (PA lipophiles dans les glycérides, PA plus hydrophiles dans les polyéthylèneglycols).

En outre, contrairement à ce qui est observé pour de nombreux vecteurs de médicaments, la stabilité des suspensions de cellisomes de l'invention s'étend sur plusieurs dizaines de mois sans nécessiter aucune étape de lyophilisation comme dans le cas des liposomes par exemple. La stabilisation des cellisomes de l'invention est assurée par un mélange de tensio-actifs contenant des phospholipides (*e.g.* Phospholipon® 90G) et des mélanges de tensioactifs non-ioniques dont certains de type « hydrophobe - hydrophile - hydrophobe » de HLB>11 selon l'échelle de Griffin. Ces tensioactifs tri-séquencés peuvent par exemple appartenir à la famille des macrogol-glycérides (*e.g.* Gelucire® 50/13) ou des acides gras polyoxyéthylénés (*e.g.* Polyoxyéthylène 40 stéarate). Ce mélange de tensioactifs a préalablement montré son intérêt dans la stabilisation à très long terme (plus de 4 ans) de nanoparticules de beurre de cacao et la très faible toxicité de ces dernières a été montrée sur un test colorimétrique au sel de tetrazolium (MTT). Le mélange et le traitement de ces excipients selon le protocole décrit ci-après aboutit à la formation des cellisomes de l'invention, de préférence dans le cas d'une phase lipidique à base de linoleoyl polyoxylglycérides et/ou oleyl polyoxylglycérides (Labrafil®), clairement différente d'une image de nanoémulsion typique (NE).

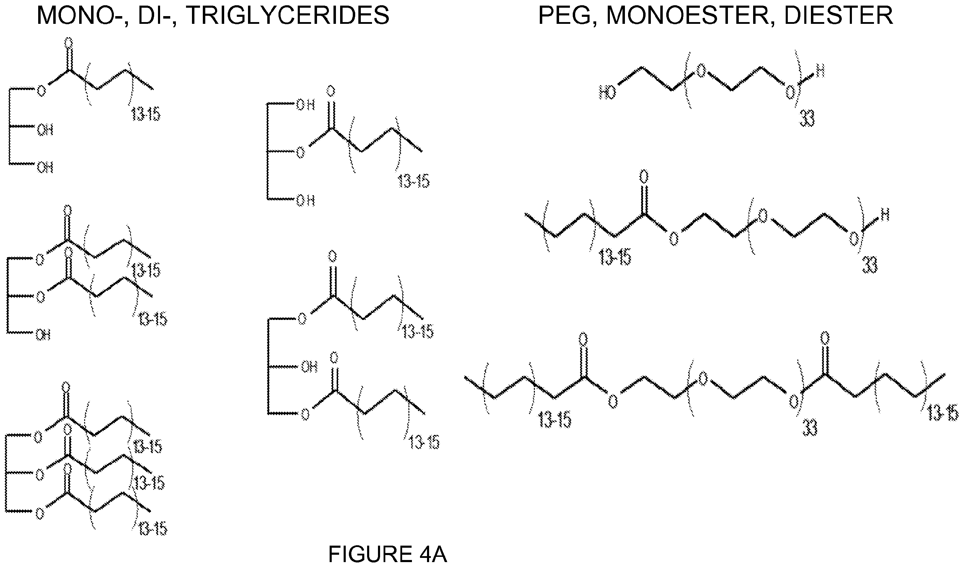

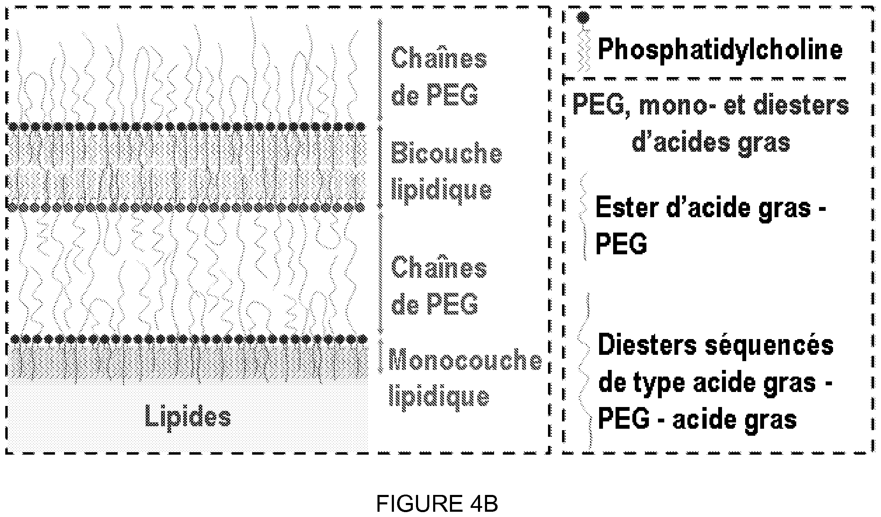

Par ailleurs, des mesures d'épaisseur de la couche sombre extérieure, donc dense aux électrons, suggèrent fortement un premier compartiment lipophile (matrice ou coeur lipidique) en partie enrobé par un second compartiment hydrophile délimité par une bicouche phospholipidique (bicouche). En outre, il ressort de l'étude de la structure des cellisomes de l'invention que ledit premier compartiment est en partie ancré audit second compartiment du fait de l'utilisation de tensioactifs non-ioniques adaptés tels que ceux décrits ci-dessus. Sans se trouver limité par cette explication, il semblerait que la clé de voûte de l'architecture des cellisomes de l'invention repose sur l'utilisation d'un mélange tensioactif non ionique hydrophile contenant au moins un dérivé tri-segmenté de type « hydrophobe - hydrophile - hydrophobe ». Les segments hydrophobes pourront être constitués d'alcools gras, d'acides gras, de glycérides, de cholestérol ou de tout autre groupement affin pour les membranes phospholipidiques. La partie hydrophile sera constituée de polymères de type polyoxyéthylène, polypropylène, polysaccharide ou tout type de polymère capable de générer, en association avec les segments hydrophobes, des tensioactifs de HLB >11. Le HLB est ici défini selon l'échelle de Griffin. Ces tensioactifs pourront par exemple appartenir à la famille des polyoxylglycérides ou macrogol-glycérides, des esters d'acides gras polyoxyéthylénés ou des alkyl éthers de polyoxyéthylène, de préférence seront le Gelucire 50/13 ou le polyoxyéthylène (40) stéarate. Les esters d'acide gras polyoxyéthylénés sont des mélanges d'acide gras estérifiés par une chaine hydrophile de poly(oxyéthylèneglycol), et contiennent un mélange, en proportion variable, de dérivés séquencés à deux blocs de type acide gras - PEG (monoesters) et de dérivés séquencés à trois blocs de type acide gras - PEG - acide gras (diesters). Les macrogol-glycérides contiennent en plus des glycérides. En particulier, le Gelucire® 50-13 est un mélange de glycérides et d'acide gras estérifiés par une chaine hydrophile de poly(oxyéthylèneglycol) de 1500g/mol, qui contient environ 40% de dérivés séquencés à trois blocs de type acide gras - PEG - acide gras (figure 4A). Dans le système proposé, la fraction de diesters est très certainement le point d'ancrage entre la bicouche et la partie lipidique (figure 4B). En effet, il est connu de l'art que l'incorporation de phospholipides dans une formulation de type particules lipidiques aboutit généralement à des préparations hétérogènes contenant deux types d'objets, des nanoparticules d'une part et des liposomes d'autre part. Un même résultat a été obtenu en appliquant le protocole expérimental de l'invention en l'absence des tensioactifs non-ioniques de type « hydrophobe - hydrophile - hydrophobe » de HLB>11 selon l'échelle de Griffin (données non représentées). Dans le système proposé, la fraction de ces tensioactifs est très certainement le point d'ancrage entre la bicouche et la partie lipidique (figure 4B).

La présente invention a donc pour objet des nanoparticules lipidiques multicompartimentées (ou cellisomes) ayant un diamètre moyen d'environ 10 à 500 nm, caractérisées en ce qu'elles comprennent un premier compartiment lipophile en partie enrobé par un second compartiment hydrophile délimité par une bicouche phospholipidique, ledit premier compartiment étant en partie ancré audit second compartiment.

Selon un mode de réalisation particulier de la présente invention, les cellisomes de l'invention ont un indice de polydispersité (*i.e.* la distribution de taille d'une population de particules) d'environ 5 à 15 %, de préférence de 10%.

De préférence les cellisomes de l'invention ont un diamètre moyen d'environ 100 à 250 nm.

Selon un mode de réalisation particulier de l'invention, le compartiment lipophile des cellisomes de l'invention est constitué de lipides dans l'état liquide ou semi-solide à température ambiante. De préférence, ledit compartiment lipophile est essentiellement constitué d'un ester d'acide gras éventuellement mélangé avec un glycéride.

De préférence ledit ester d'acide gras est choisi dans le groupe consistant en des mélanges de mono-, di- esters de polyéthylène glycols dont la masse molaire de la partie hydrophile varie de 100 à 700 g/mol.

De préférence le glycéride est choisi dans le groupe consistant en des mélanges de mono-, di- et tri-esters de glycérol liquide ou solide à température ambiante.

Selon un mode de réalisation particulier de l'invention, la bicouche phospholipidique des cellisomes de l'invention est essentiellement constituée de phospholipide et de tensio-actif hydrophile non ionique.

De préférence ledit phospholipide est choisi dans le groupe consistant en des mélanges de phospholipides essentiellement zwitterioniques.

Ledit tensio-actif non ionique est choisi dans le groupe consistant en un mélange tensioactif non ionique hydrophile contenant au moins un dérivé tri-segmenté de type « hydrophobe - hydrophile - hydrophobe ». Les segments hydrophobes pourront être constitués d'alcools gras, d'acides gras, de glycérides, de cholestérol ou de tout autre groupement affin pour les membranes phospholipidiques. La partie hydrophile sera constituée de polymères de type polyoxyéthylène, polypropylène, polysaccharide ou tout type de polymère capable de générer, en association avec les segments hydrophobes, des tensioactifs de HLB >11. Le HLB est ici défini selon l'échelle de Griffin. Ces tensioactifs pourront par exemple appartenir à la famille des polyoxylglycérides ou macrogol-glycérides, des esters d'acides gras polyoxyéthylénés ou des alkyl éthers de polyoxyéthylène, de préférence seront le Gelucire 50/13 ou le polyoxyéthylène (40) stéarate.

La présente invention a également pour objet des nanoparticules lipidiques multicompartimentées (ou cellisomes) de l'invention comprenant en outre au moins une molécule d'intérêt.

De préférence la molécule d'intérêt est choisie parmi un acide nucléique, une protéine, un polysaccharide, une petite molécule ou toute autre molécule d'intérêt pharmaceutique, cosmétique et/ou agro-alimentaire.

On entend par « petite molécule » au sens de la présente invention, par exemple, des molécules présentant une masse moléculaire de 60 à 5000 Da, de préférence de 40 à 3000 Da, tout préférentiellement < 2000 Da.

La présente invention a également pour objet une composition pharmaceutique comprenant une suspension de nanoparticules lipidiques multicompartimentées (ou cellisomes) de l'invention dans un excipient pharmaceutiquement acceptable.

La présente invention a également pour objet un procédé de préparation de nanoparticules lipidiques multicompartimentées (ou cellisomes) de l'invention comprenant les étapes suivantes :
- préparation d'une émulsion huile/eau comprenant au moins un lipide, un phospholipide, un tensio-actif hydrophile non ionique, et éventuellement au moins une molécule d'intérêt ;
- homogénéisation de ladite émulsion sous haute pression.

Selon un mode de réalisation particulier de l'invention, ledit procédé de l'invention comprend en outre une étape de chauffage de l'émulsion à une température de 30 à 80°C, de préférence à 70°C, avant son homogénéisation.

De préférence ledit lipide est choisi dans le groupe consistant en des mélanges de mono- et di-esters de polyéthylène glycols dont la masse molaire de la partie hydrophile varie de 100 à 700 g/mol.

De préférence ledit phospholipide est choisi dans le groupe consistant en des mélanges de phospholipides essentiellement zwitterioniques.

De préférence ledit tensio-actif hydrophile non ionique est choisi dans le groupe consistant en la famille des polyoxylglycérides ou macrogol-glycérides, des esters d'acides gras polyoxyéthylénés ou des alkyl éthers de polyoxyéthylène, de préférence le Gelucire® 50/13 ou le polyoxyéthylène (40) stéarate.

De préférence l'émulsion huile/eau du procédé de l'invention comprend :
- 5-30% de lipides, de préférence du Labrafil® M2125CS ou M1944CS;
- 0,5-5% de phospholipides, de préférence du Phospholipon 90G® ;
- 0,5-5% de macrogol-glycérides ou d'esters d'acide gras polyoxyéthylénés, de préférence du Gélucire® 50-13 ou du polyoxyéthylène (40) stéarate ;
- Eau, qsp pour 100g.

Les « % » s'entendent en % de la masse totale de l'émulsion.

La présente invention a également pour objet les nanoparticules lipidiques multicompartimentées (ou cellisomes) de l'invention pour une utilisation comme vecteur pour l'administration d'une molécule d'intérêt.

La présente invention a également pour objet les nanoparticules lipidiques multicompartimentées (ou cellisomes) de l'invention pour une utilisation comme médicament, de préférence un médicament administré par voie injectable, orale, nasale ou cutanée.

D'autres avantages pourront encore apparaître à l'homme du métier à la lecture des exemples ci-dessous, illustrés par les figures annexées, donnés à titre illustratif.

### Brève description des figures

- La figure 1 représente des images de microscopie électronique de structures nommées « sacs à main » ou « hand-bags » obtenus avec différentes conditions opératoires. Quelque soient ces dernières, la proportion de « sacs à main » reste minoritaire.
- La figure 2 représente les descriptions schématiques et images en cryo-microscopie électronique par transmission (cryo-TEM) de liposomes (A) et de particules lipidiques (B).
- La figure 3 montre une image en cryo-TEM des nanoparticules lipidiques multicompartimentées de l'invention.
- La figure 4 représente les constituants du Gelucire® 50-13 (A), et un schéma de la supra-structure membranaire des nanoparticules lipidiques multicompartimentées de l'invention (B).
- La figure 5 représente un schéma de préparation des nanoparticules lipidiques multicompartimentées de l'invention.
- La figure 6 représente le suivi sur 20 mois de la taille des nanoparticules lipidiques multicompartimentées et de leur polydispersité pour le Labrafil® M 2125 CS

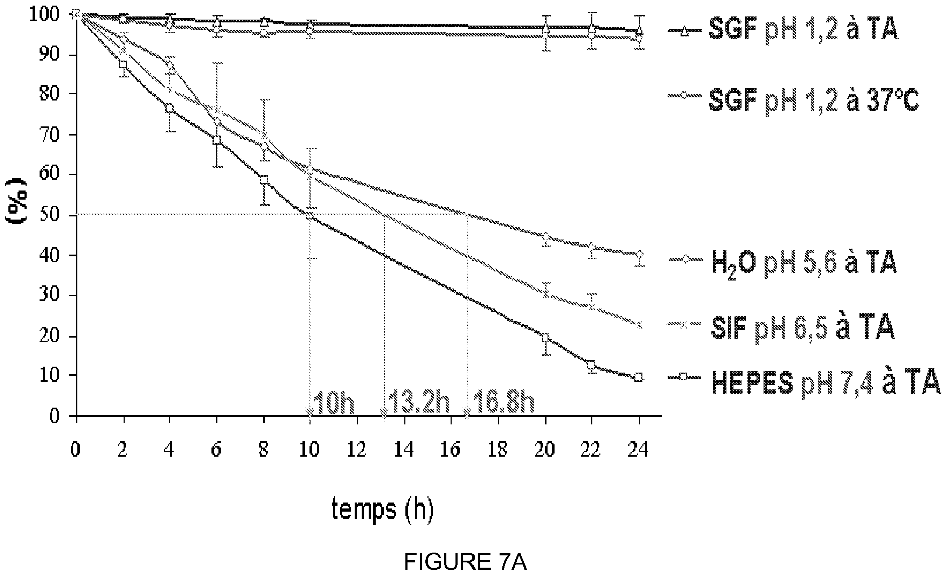

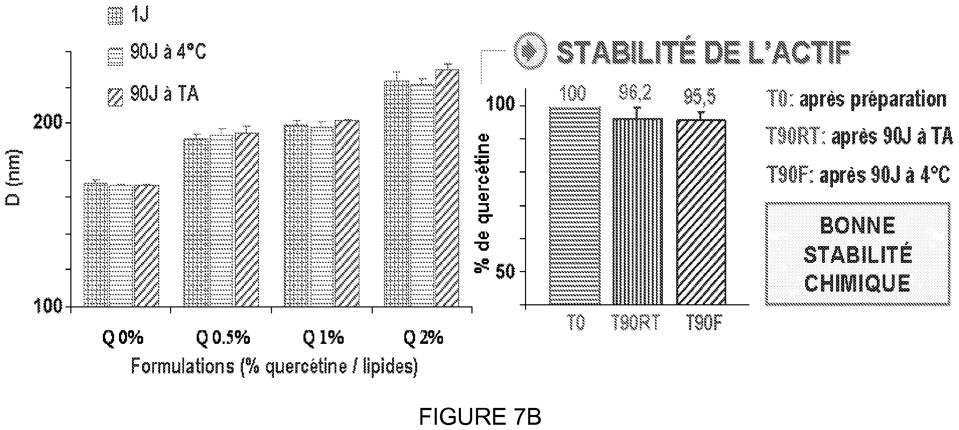

- La figure 7 représente l'étude de la stabilité de la quercétine solubilisée en milieu aqueux [H₂O à température ambiante ; milieu aqueux à pH7,4 à température ambiante ; milieu gastrique simulé (SGF) à température ambiante ; milieu gastrique simulé (SGF) à 37°C ; milieu intestinal reconstitué (SIF) à température ambiante] (A), et de la stabilité chimique de différentes concentrations (1, 2, 3, 4 et 5%) de quercétine encapsulée dans les nanoparticules lipidiques multicompartimentées de l'invention (B, droite) ; la stabilité physique des particules est représentée sur la partie gauche de la figure 7B.

### EXEMPLES

### Exemple 1 : Obtention de nanoparticules lipidiques multicompartimentées (ou cellisomes)

### Préparation

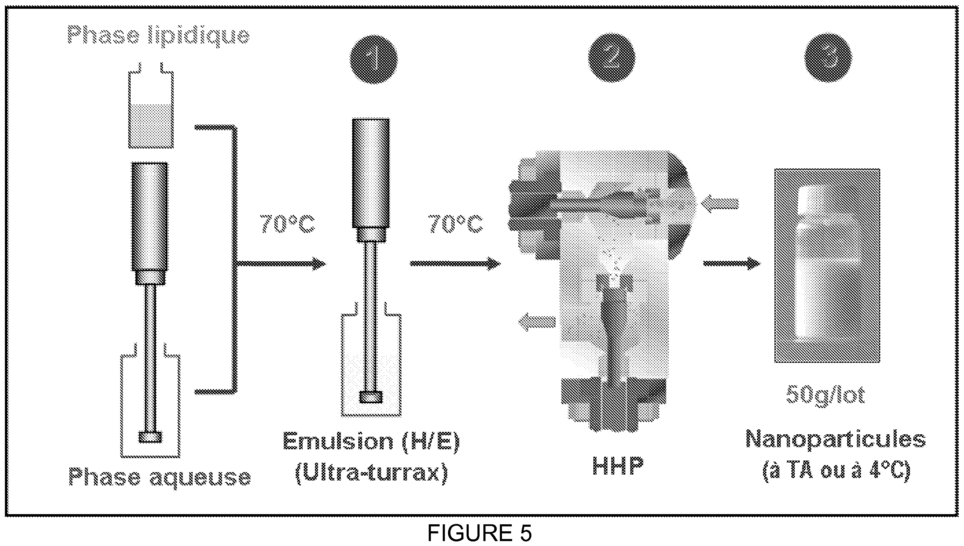

La préparation des nanoparticules lipidiques multicompartimentées est schématisée dans la figure 5.

Les nanodispersions ont été préparées à l'aide d'un homogénéisateur à 2 étages (APV 2000, Invensys, Albertslund, Danemark) équipé d'un système de chauffage et associé à un Ultra-turrax T25 (Janke & Kunkel GMBH & Co.KG, IKA®-Labotechnik, Allemagne) pour la préparation de l'émulsion primaire.

Pour chaque lot, 50 g de nanodispersions ont été préparés selon le protocole incluant les trois étapes suivantes:
***Dispersion** :* Tout d'abord, les lipides et les tensioactifs (Gélucire® 50/13 et Phospholipon® 90G) ont été pesés et introduits dans des flacons de 20 ml et 100 ml, respectivement. La quantité d'eau nécessaire a été ajoutée dans le flacon contenant les tensioactifs puis la suspension de tensioactifs a été préparée à 70°C sous agitation mécanique (Ultra-turrax T25, Janke & Kunkel GMBH & Co.KG, IKA®-Labotechnik, Allemagne), à la vitesse de 9500 rpm pendant 5 min. Enfin, le mélange lipidique, également chauffé à 70°C, a été ajouté dans la phase aqueuse et agité par Ultra-turrax à la vitesse de 13500 rpm pendant 5 min, pour obtenir une pré-dispersion (H/E).
***Diminution et homogénéisation de la taille des particules par HHP :*** La pré-dispersion obtenue a été rapidement introduite dans l'homogénéisateur à deux étages (APV 2000, Invensys, Albertslund, Danemark) chauffé au préalable à 70°C. Elle y a subi plusieurs cycles d'homogénéisation continue pendant 5 minutes, sous un double effet de pression (la pression du 1^{er} étage est de 600 bar et celle du 2^{nd} étage est de 200 bar), pour former une nanoémulsion (H/E) à chaud.
***Refroidissement des particules*** : Les nanodispersions ont été ensuite maintenues à température ambiante et à 4°C.

### Composition

Les nanoparticules lipidiques multicompartimentées obtenues par le procédé de préparation décrit ci-dessus comprennent à titre d'exemple :

| | |
|---|---|
| Lipides (Labrafil®) | 5 à 20% |
| Phospholipides (Phospholipon 90G) | 1% |
| Macrogol-glycérides (Gelucire® 50-13) | 2% |
| Eau (qsp) | 100g |

### Stabilité des nanoparticules lipidiques multicompartimentées

Le stockage à température ambiante des nanoparticules lipidiques multicompartimentées en suspension dans l'eau a été suivi sur plusieurs mois, au regard de leur taille et de leur polydispersité. Ce suivi a été montré en exemple pour le Labrafil® M 2125 CS. L'écart-type sur le diamètre moyen (n=3) généralement masqué par le symbole, a confirmé la bonne reproductibilité du procédé. Le diamètre de nanoparticule inférieur à 200 nm est adapté à une administration intra-veineuse. La polydispersité inférieure à 0,2 traduit une distribution étroite des tailles autour de la moyenne.

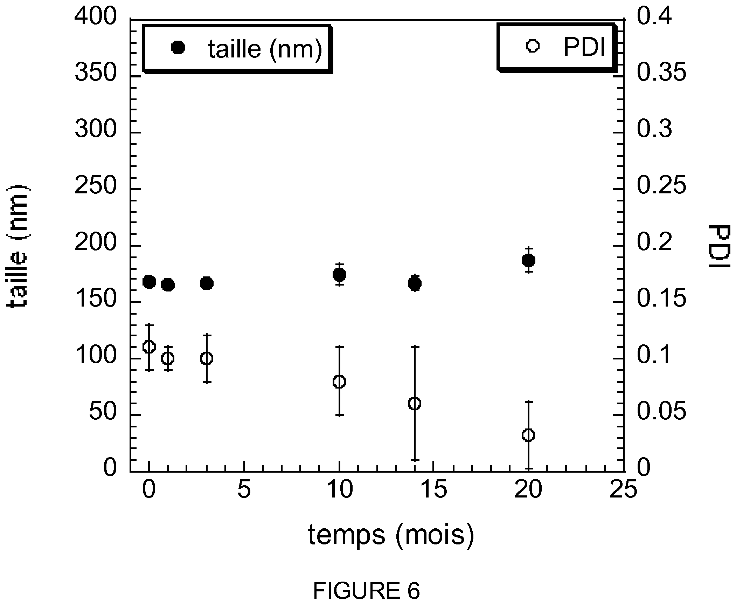

En suspension dans l'eau, les nanoparticules lipidiques multicompartimentées les plus anciennes ont conservé un diamètre (170-180 nm) et une polydispersité (∼0,1) identiques pendant plus de 20 mois (figure 6).

### Exemple 2: Nanoparticules lipidiques multicompartimentées (ou cellisomes) comme vecteurs de quercétine

### Préparation des nanoparticules lipidiques multicompartimentées comme vecteurs de principes actifs

Les nanoparticules lipidiques multicompartimentées obtenues par le protocole ci-dessus ont été testées quant à leur capacité à encapsuler des principes actifs pharmaceutiques.

Pour ce faire, à titre d'exemple, des études ont été menées sur l'encapsulation de la quercétine. Ce principe actif présente le double inconvénient d'être très peu soluble dans l'eau (∼0.4 µg/ml) et rapidement hydrolysé dans certaines conditions de pH. Les bénéfices de l'encapsulation sur ces deux points ont donc été étudiés.

Les particules ont été préparées suivant le protocole décrit précédemment, en solubilisant à saturation le principe actif dans la phase lipidique avant son émulsification dans la phase aqueuse contenant les tensioactifs. Le principe actif non encapsulé a été séparé par ultracentrifugation (LE-80K Ultracentrifuge Optima, 30000 rpm, 1h, 4°C) puis la fraction encapsulée a été dosée dans le surnageant, contenant les nanoparticules, par chromatographie liquide haute performance (CLHP) selon une méthode développée au laboratoire. A savoir, la quercétine a été dosée à l'aide d'un appareillage CLHP de chez Waters™, équipé d'un détecteur UV (Waters™ 2487 Dual λ Absorbance Detector), d'une pompe (Waters™ 1525 Binary CLHP Pump), d'un injecteur associé à un passeur d'échantillon automatisé (Waters™ 717 plus Autosampler) et d'un dégazeur en ligne (Waters™ In-Line Degasser AF). La séparation a été effectuée sur une colonne (Modulo-cart QK 3 C18-2 colonnes, 150 mm x 3 mm, Interchim) à 25°C avec une phase mobile acétonitrile - eau - acide trifluoroacétique (30:70:0.1, v/v). La longueur d'onde du détecteur a été fixée à 371 nm. Le débit était de 0,5 ml/min et le volume injecté de 20 µl. La gamme d'étalonnage établie à partir de trois solutions mères indépendantes et contenant des particules lipidiques blanches possédait un coefficient de corrélation (r²) de 0.9998 et une zone de linéarité large de 50 à 5000 ng/ml. Ces chiffres ont confirmé que la spécificité, la sensibilité et la précision de la méthode étaient élevées dans les conditions de chromatographie déterminées. Avant l'analyse CLHP, tous les échantillons ont été dissous dans le méthanol à des concentrations comprises dans la gamme linéaire, puis filtrés à travers une membrane de porosité moyenne 0,2 µm (Minisart® High-Flow Hydrophilic 16532K, Sartorius Stedim Biotech GmbH, Goettingen, Allemagne). La méthode CLHP mise au point permet de séparer le PA et les excipients, et donc de déterminer directement la teneur en PA encapsulé dans les particules lipidiques, ainsi que la stabilité et les cinétiques de libération dans les différents milieux de dissolution.

La charge maximale en principe actif dans la suspension est d'environ 1% (soit 5.10³ fois sa solubilité aqueuse) avec un rendement d'encapsulation d'environ 96% dans les conditions optimum.

En outre, il est apparu que la présence de PEG à la surface des nanoparticules leur permet *a priori* de bénéficier de la propriété de furtivité (échappement à la capture par le système réticulo-endothélial) largement décrite pour les liposomes « pegylés » et primordiale pour leur administration *in vivo.*

### Stabilité de la quercétine libre ou encapsulée

La stabilité de la quercétine, hydrolysée en milieu aqueux et encapsulée dans les nanoparticules lipidiques multicompatimentées de l'invention, a été testée.

Pour ce faire, la stabilité de la quercétine dans différents milieux a été quantifiée par dosage CLHP. Les différents milieux considérés ont été le milieu gastrique simulé (SGF - *US Pharmacopeia*) à température ambiante et à 37°C, le milieu intestinal simulé (SIF - *US Pharmacopeia*), un tampon HEPES 10 mM (pH 7,4) et l'eau à température ambiante. Des prélèvements sur 24h ont notamment montré que dans de l'eau à température ambiante, environ 50% de la quercétine est dégradée en 17h (figure 7A) alors que cette molécule est plus stable dans les milieux plus acides.

En revanche, après encapsulation, et stockage sous forme de suspension dans l'eau, le dosage après ultracentrifugation des nanoparticules a montré qu'environ 100% de la quercétine était retrouvée après 90 jours de conservation (figure 7B droit). Par ailleurs, les particules encapsulant le principe actif sont restées physiquement stables au moins 90 jours, à température ambiante comme à 4°C (figure 7B gauche).

### Exemple 3 : Obtention de nanoparticules lipidiques multicompartimentées (ou cellisomes)

D'autres cellisomes ont été obtenus selon le protocole précédemment décrit à partir d'excipients différents.

Par exemple, la partie lipidique a été constituée de linoleoyl macrogol-6 glycérides (European Pharmacopea), d'oleoyl macrogol-6 glycérides (EP) ou de Propylène glycol monolaurate (EP); le phospholipide a été de la dipalmitoyl-phosphatidylcholine ; le tensioactif non-ionique a été du stéaroyl macrogol-32 glycérides (EP), un mélange synthétique de mono- et di-esters d'acide stéarique et de macrogol-32 (40/60 m/m) ou du polyoxyéthylene (40) stéarate.

Pour l'ensemble de ces excipients, les observations en cryo-TEM ont confirmé que les nanoparticules étaient compartimentées et de morphologie similaire à celle décrite dans la figure 3.

### Exemple 4: Obtention des nanoparticules lipidiques multicompartimentées (ou cellisomes) en lot de 10g

### Préparation

La préparation des nanoparticules lipidiques multicompartimentées est schématisée dans la figure 5.

Les nanodispersions ont été préparées à l'aide d'un homogénéisateur à 1 étage (Laboratory HPH Stansted Fluid Power Ltd, Angleterre) équipé d'un système de chauffage et associé à un Ultra-turrax IKA T10 (Janke & Kunkel GMBH & Co.KG, IKA®-Labotechnik, Allemagne) pour la préparation de la dispersion primaire.

10 g de nanodispersions ont été préparés selon le protocole incluant les trois étapes suivantes:
***Dispersion** :* Tout d'abord, les lipides et les tensioactifs (Gélucire® 50/13 et Phospholipon® 90G) ont été pesés et introduits dans des flacons de 7 ml et 20 ml, respectivement. La quantité d'eau nécessaire a été ajoutée dans le flacon contenant les tensioactifs puis la suspension de tensioactifs a été préparée à 70°C sous agitation mécanique (Ultra-turrax IKA T10, Janke & Kunkel GMBH & Co.KG, IKA®-Labotechnik, Allemagne), à la vitesse de 8500 rpm pendant 5 min. Enfin, le mélange lipidique, également chauffé à 70°C, a été ajouté dans la phase aqueuse et agité par Ultra-turrax à la vitesse de 20000 rpm pendant 5 min, pour obtenir une pré-dispersion (H/E).
***Diminution et homogénéisation de la taille des particules par HHP :*** La dispersion obtenue a été rapidement introduite dans l'homogénéisateur à 1 étage (Laboratory HPH Stansted Fluid Power Ltd, Angleterre) chauffé au préalable à 70°C. Elle y a subi 4 cycles d'homogénéisation sous une pression de 1000 bars.
***Refroidissement des particules*** : Les nanodispersions ont été ensuite maintenues à température ambiante.

### Composition

Les nanoparticules lipidiques multicompartimentées obtenues par le procédé de préparation décrit ci-dessus comprennent (% en masse) :

| | |
|---|---|
| Labrafil®M2125CS | 20% |
| Phospholipon 90G | 1% |
| Gelucire® 50-13 | 2% |
| Eau (qsp) | 10g |

### Analyses de taille et de morphologie

A la suite de ce procédé de préparation, les nanoparticules ont un diamètre hydrodynamique de 228 ± 2 nm. Par ailleurs, les observations en cryo-TEM confirment que les nanoparticules sont compartimentées et de morphologie similaire à celle décrite dans la figure 3.

### Exemple 5 : Nanoparticules lipidiques multicompartimentées (ou cellisomes) comme vecteurs de molécules d'intérêt pharmaceutique et/ou cosmétique en lot de 10g

Les nanoparticules lipidiques multicompartimentées ont été préparées selon le protocole décrit dans l'exemple 4, et testées quant à leur capacité à encapsuler différents principes actifs.

En vue d'applications cutanées, des molécules d'intérêt dermo-cosmétique ont été sélectionnées en fonction de leur coefficient de partage octanol-eau calculé (LogP) et de leur masse molaire puis incorporées dans la formulation. Les trois molécules ont été : la caféine (anticellulite ; logP=-0,55 ; 194 g/mol), le chloroxylénol (antiseptique ; logP=3,30 ; 156 g/mol) et l'irgasan (antiseptique ; logP=4,98 ; 289 g/mol).

Ces molécules d'intérêt ont été solubilisées, soit dans la phase aqueuse pour les hydrophiles, soit dans la phase lipidique pour les lipophiles, avant le mélange par ultra-turrax dans le protocole décrit dans l'exemple 3. Compte tenu de leurs solubilités dans les différents milieux constitutifs des nanoparticules compartimentées, la caféine a été introduite dans la phase aqueuse alors que le chloroxylénol et l'irgasan ont été incorporés dans la phase lipidique.

La concentration finale en chacun des composés d'intérêt dans la dispersion de nanoparticules multicompartimentées est de 1% en masse.

### Analyses de taille et de morphologie

A la suite de ce procédé de préparation, les nanoparticules contenant la caféine ont présenté un diamètre hydrodynamique de 223 ± 3 nm qui est resté stable au moins 12 jours à 25°C, les nanoparticules contenant l'irgasan ont présenté un diamètre hydrodynamique de 224 ± 4 nm qui est resté stable au moins 12 jours à 25°C, et les nanoparticules contenant le chloroxylénol ont présenté un diamètre hydrodynamique de 245 ± 2 nm qui est resté stable au moins 1 mois à 25°C.

Pour chacune des molécules incorporées, les observations en cryo-TEM ont confirmé que les nanoparticules sont compartimentées et de morphologie similaire à celle décrite dans la figure 3.

### Exemple 6: Obtention d'un gel de nanoparticules lipidiques multicompartimentées (ou cellisome)

Les nanoparticules lipidiques multicompartimentées ont été préparées selon le protocole décrit dans l'exemple 4.

A l'issue de cette fabrication, les nanoparticules compartimentées produites ont été mélangées avec un gel préformé à 2% en Carbopol® 974 NF neutralisé par de la triéthanolamine. Concentrés à 0,2% et 0,4% en Carbopol® 974 NF et contenant respectivement 20,7% et 18,4% (en masse) d'excipients lipidiques, les gels finaux gardent le caractère rhéofluidifiant des gels de Carbopol® 974 NF sans nanoparticules.

Des mesures de diamètres hydrodynamiques et des observations en cryo-TEM ont montré que le mélange avec le Carbopol® 974 NF n'altérait ni la taille des nanoparticules, ni leur morphologie compartimentée même après 5 mois de stockage à 25°C.

### Exemple 7: Obtention d'un lot de 1 kg de nanoparticules lipidiques multicompartimentées (ou cellisome)

### Préparation

La préparation des nanoparticules lipidiques multicompartimentées est schématisée dans la figure 5.

Les nanodispersions ont été préparées à l'aide d'un homogénéisateur à 2 étages (APV 2000, Invensys, Albertslund, Danemark) équipé d'un système de chauffage et associé à un Ultra-turrax T18 Basic (Janke & Kunkel GMBH & Co.KG, IKA®-Labotechnik, Allemagne) pour la préparation de la dispersion primaire.

1kg de nanodispersions a été préparé selon le protocole incluant les trois étapes suivantes:
***Dispersion** :* Tout d'abord, les lipides et les tensioactifs (Gélucire® 50/13 et Phospholipon® 90G) ont été pesés et introduits dans des béchers de 200 ml et 1000 ml, respectivement. La quantité d'eau nécessaire a été ajoutée dans le bécher contenant les tensioactifs puis la suspension de tensioactifs a été préparée à 70°C sous agitation mécanique (Ultra-turrax T18 Basic, Janke & Kunkel GMBH & Co.KG, IKA®-Labotechnik, Allemagne), à la vitesse de 11000 rpm pendant 15 min. Enfin, le mélange lipidique, également chauffé à 70°C, a été ajouté dans la phase aqueuse et agité par Ultra-turrax à la vitesse de 20000 rpm pendant 15 min, pour obtenir une pré-dispersion (H/E).
***Diminution et homogénéisation de la taille des particules par HHP :*** La dispersion obtenue a été introduite dans l'homogénéisateur à deux étages (APV 2000, Invensys, Albertslund, Danemark) chauffé au préalable à 70°C et maintenue sous agitation mécanique à l'aide d'un mélangeur Rayneri 1144 (800 rpm). Elle y a subi plusieurs cycles d'homogénéisation continue pendant 15 minutes, sous un double effet de pression (la pression du 1^{er} étage est de 600 bar et celle du 2^{nd} étage est de 200 bar), pour former une nanodispersion (H/E) à chaud.
***Refroidissement des particules*** : Les nanodispersions ont été ensuite conditionnées en flacon de 20 ml et maintenues à température ambiante.

### Composition

Les nanoparticules lipidiques multicompartimentées obtenues par le procédé de préparation décrit ci-dessus comprennent (% en masse) :

| | |
|---|---|
| Labrafil®M2125CS | 20% |
| Phospholipon 90G | 1% |
| Gelucire® 50-13 | 2% |
| Eau (qsp) | 1 kg |

### Analyses de taille et de morphologie

A la suite de ce procédé de préparation, les observations en cryo-TEM ont confirmé que les nanoparticules étaient compartimentées et de morphologie similaire à celle décrite dans la figure 3.

### Listes des références

1. Meure et al., Aaps Pharmscitech, 9 : 798-809, 2008
2. Storm et Crommelin, Pharmaceutical Science & Technology Today, 1 : 19-31, 1998
3. Bunjes, Current Opinion in Colloid & Interface Science, 16(5) : 405-411, 2011
4. Harde et al., Expert Opinion on Drug Delivery, 8(11): 1407-1424, 2011
5. Harms et al., Journal of Drug Delivery Science and Technology, 21(1) : 89-99, 2011
6. Joshi et Muller, European Journal of Pharmaceutics and Biopharmaceutics, 71 : 161-172, 2009
7. Muller et al., Current Drug Discovery technologies, 8(3) : 207-227, 2011
8. Pardeike et al., International Journal of Pharmaceutics, 366 : 170-184, 2009
9. Souto et Doktorovova, Methods in Enzymology, 464 : 105-129, 2009
10. Demande Internationale WO 01/64328
11. Huynh et al., Journal of Pharmaceutics, 379 : 201-209, 2009
12. Texeira et al., Pharmaceutical Research, 17 : 1329-1332, 2000
13. Brevet US 5,576,016
14. Gupta et al., Journal of Drug Targeting, 15 : 437-444, 2007
15. Gupta et Vyas, Journal of Drug Targeting, 15 : 206-217, 2007
16. Kretschmar et al., Mycoses, 44 : 281-286, 2001
17. Paliwal et al., International Journal of Pharmaceutics, 380 : 181-188, 2009
18. Wu et al., Journal of Immunology, 185(6) : 3401-3407, 2010

## Revendications

1. Nanoparticules lipidiques multicompartimentées anisotropes ayant un diamètre moyen de 10 à 500 nm, **caractérisées en ce qu'**elles comprennent un premier compartiment lipophile qui n'est qu'en partie enrobé par un second compartiment hydrophile délimité par une bicouche phospholipidique, ledit premier compartiment n'étant qu'en partie ancré audit second compartiment,
et **caractérisées en ce que** lesdites nanoparticules sont stabilisées par un mélange de tensioactifs comprenant (a) des phospholipides, et (b) un mélange de tensioactifs non-ioniques comprenant au moins un dérivé tri-segmenté de type « hydrophobe-hydrophile-huydrophobe » de HLB supérieur à 11 selon l'échelle de Griffin.

2. Nanoparticules selon la revendication 1, où l'indice de polydispersité est de 5 à 15 %.

3. Nanoparticules selon la revendication 1 ou 2, où le diamètre moyen est de 100 à 250 nm.

4. Nanoparticules selon l'une quelconque des revendications 1 à 3 où le compartiment lipophile est constitué de lipides dans l'état liquide ou semi-solide à 25°C.

5. Nanoparticules selon l'une quelconque des revendications 1 à 4, où le compartiment lipophile comprend un ester d'acide gras éventuellement mélangé avec un glycéride.

6. Nanoparticules selon la revendication 5, où ledit ester d'acide gras est choisi dans le groupe consistant en des mélanges de mono-, di- esters de polyéthylène glycols dont la masse molaire de la partie hydrophile varie de 100 à 700 g/mol.

7. Nanoparticules selon la revendication 5, où le glycéride est choisi dans le groupe consistant en des mélanges de mono-, di- et tri-esters de glycérol liquide ou solide à 25°C.

8. Nanoparticules selon l'une quelconque des revendications 1 à 7, où la bicouche phospholipidique comprend au moins un phospholipide et un tensioactif hydrophile non ionique.

9. Nanoparticules selon la revendication 8, où ledit phospholipide est choisi dans le groupe consistant en des mélanges de phospholipides zwitterioniques.

10. Nanoparticules selon la revendication 8, où ledit tensioactif hydrophile non ionique est choisi dans le groupe consistant en les polyoxylglycérides ou macrogol-glycérides, les esters d'acides gras polyoxyéthylénés et les alkyl éthers de polyoxyéthylène

11. Nanoparticules selon l'une quelconque des revendications 1 à 10, comprenant en outre au moins une molécule d'intérêt.

12. Nanoparticules selon la revendication 11, où la molécule d'intérêt est choisie parmi un acide nucléique, une protéine, un polysaccharide et une molécule de 40 à 2000 Da.

13. Composition pharmaceutique comprenant une suspension de nanoparticules telles que définies dans l'une quelconque des revendications 1 à 12 dans un excipient pharmaceutiquement acceptable.

14. Procédé de préparation de nanoparticules lipidiques multicompartimentées selon la revendication 1 comprenant les étapes suivantes :
- préparation d'une émulsion huile/eau comprenant au moins un lipide, des phospholipides, un mélange de tensio-actifs non ioniques comprenant au moins un dérivé trisegmenté de type « hydrophobe-hydrophile-hydrophobe » de HLB supérieur à 11 selon l'échelle d Griffin, et éventuellement au moins une molécule d'intérêt ;
- homogénéisation de ladite émulsion sous haute pression.

15. Procédé selon la revendication 14, comprenant en outre une étape de chauffage de l'émulsion à une température de 30 à 80°C avant son homogénéisation.

16. Procédé selon la revendication 14, où ledit lipide est choisi dans le groupe consistant en des mélanges de mono- et di-esters de polyéthylène glycols dont la masse molaire de la partie hydrophile varie de 100 à 700 g/mol.

17. Procédé selon la revendication 14, où ledit phospholipide est choisi dans le groupe consistant en des mélanges de phospholipides zwitterioniques.

18. Procédé selon la revendication 14, où ledit tensio-actif hydrophile non ionique est choisi dans le groupe consistant en les polyoxylglycérides ou macrogol-glycérides, les esters d'acides gras polyoxyéthylénés et les alkyl éthers de polyoxyéthylène.

19. Procédé selon l'une quelconque des revendications 14 à 18, où l'émulsion huile/eau comprend :
- 5-30% en masse de lipides ;
- 0,5-5% en masse de phospholipides ;
- 0,5-5% en masse de polyoxylglycérides ou macrogol-glycérides, d'esters d'acides gras polyoxyéthylénés ou d'alkyl éthers de polyoxyéthylène, de préférence du polyoxyéthylène (40) stéarate.

20. Nanoparticules telles que définies dans l'une quelconque des revendications 1 à 10 pour une utilisation comme vecteur pour l'administration d'une molécule d'intérêt.

21. Nanoparticules telles que définies dans la revendication 11 ou 12 pour une utilisation comme médicament, de préférence un médicament administré par voie injectable, orale, cutanée ou nasale.

## Patentansprüche

1. Anisotrope, mehrfach unterteilte Lipid-Nanopartikel, die einen mittleren Durchmesser von 10 bis 500 nm besitzen, **dadurch gekennzeichnet, dass** sie einen ersten lipophilen Anteil enthalten, der nur ein Teil ist, der von einem zweiten hydrophilen Anteil umhüllt ist, der durch eine Phospholipid-Doppelschicht begrenzt ist, wobei der erste Anteil nur ein an dem zweiten Anteil verankerter Teil ist,
und **dadurch gekennzeichnet, dass** die Nanopartikel durch ein grenzflächenaktives Gemisch stabilisiert sind, das (a) Phospholipide und (b) ein Gemisch aus nicht ionischen Tensiden enthält, das wenigstens ein dreifach segmentiertes Derivat des Typs "hydrophob-hydrophil-hydrophob" von HLB größer als 11 auf der Griffin-Skala enthält.

2. Nanopartikel nach Anspruch 1, wobei der Polydispersitätsindex im Bereich von 5 bis 15 % liegt.

3. Nanopartikel nach Anspruch 1 oder 2, wobei der mittlere Durchmesser im Bereich von 100 bis 250 nm liegt.

4. Nanopartikel nach einem der Ansprüche 1 bis 3, wobei der lipophile Anteil aus Lipiden in einem bei 25 °C flüssigen oder halbfesten Zustand gebildet ist.

5. Nanopartikel nach einem der Ansprüche 1 bis 4, wobei der lipophile Anteil einen Fettsäureester, der eventuell mit Glycerid gemischt ist, enthält.

6. Nanopartikel nach Anspruch 5, wobei der Fettsäureester aus der Gruppe gewählt ist, die aus Gemischen von Mono- oder Di-Estern von Polyethylenglykolen, deren molare Masse des hydrophilen Teils zwischen 100 und 700 g/mol variiert, besteht.

7. Nanopartikel nach Anspruch 5, wobei Glycerid aus der Gruppe gewählt ist, die aus Gemischen aus Mono-, Di- und Tri-Estern von bei 25 °C flüssigem oder festem Glycerol besteht.

8. Nanopartikel nach einem der Ansprüche 1 bis 7, wobei die Phospholipid-Doppelschicht wenigstens ein Phospholipid und ein nicht ionisches hydrophiles Tensid enthält.

9. Nanopartikel nach Anspruch 8, wobei das Phospholipid aus der Gruppe gewählt ist, die aus Gemischen von zwitterionischen Phospholipiden besteht.

10. Nanopartikel nach Anspruch 8, wobei das nicht ionische hydrophile Tensid aus der Gruppe gewählt ist, die aus Polyoxylglyceriden oder Makrogol-Glyceriden, Polyoxyehtylen-Fettsäureestern und Polyoxyethylen-Alkylethern besteht.

11. Nanopartikel nach einem der Ansprüche 1 bis 10, die außerdem wenigstens ein interessierendes Molekül enthalten.

12. Nanopartikel nach Anspruch 11, wobei das interessierende Molekül aus Nukleinsäure, Protein, Polysaccharid und einem Molekül mit 40 bis 2000 Da gewählt ist.

13. Pharmazeutische Zusammensetzung, die eine Supsension von Nanopartikeln wie in einem der Ansprüche 1 bis 12 definiert in einer pharmazeutisch akzeptablen Grundmasse enthält.

14. Verfahren zum Bereitstellen von mehrfach unterteilten Lipid-Nanopartikeln nach Anspruch 1, das die folgenden Schritte umfasst:
- Bereitstellen einer Öl/Wasser-Emulsion, die wenigstens ein Lipid, Phospholipide, ein nicht ionisches grenzflächenaktives Gemisch, das wenigstens ein dreifach segmentiertes Derivat des Typs "hydrophob-hydrophil-hydrophob" von HLB größer 11 auf der Griffin-Skala besitzt, und eventuell wenigstens ein interessierendes Molekül enthält;
Homogenisieren der Emulsion unter hohem Druck.

15. Verfahren nach Anspruch 14, das außerdem einen Schritt des Erwärmens der Emulsion auf eine Temperatur von 30 bis 80 °C vor ihrer Homogenisierung umfasst.

16. Verfahren nach Anspruch 14, wobei das Lipid aus der Gruppe gewählt ist, die aus Gemischen von Mono- und Di-Estern von Polyethylenglykolen, deren molare Masse des hydrophilen Anteils zwischen 100 und 700 g/mol variiert, besteht.

17. Verfahren nach Anspruch 14, wobei das Phospholipid aus der Gruppe gewählt ist, die aus zwitterionischen Phospholipid-Gemischen besteht.

18. Verfahren nach Anspruch 14, wobei das nicht ionische hydrophile Tensid aus der Gruppe gewählt ist, die aus Polyoxylglyceriden oder Makrogol-Glyceriden, Polyoxyethylen-Fettsäureestern und Polyoxyethylen-Alkylethern besteht.

19. Verfahren nach einem der Ansprüche 14 bis 18, wobei die Öl/Wasser-Emulsion Folgendes umfasst:
- 5-30 Massen-% Lipide;
- 0,5-5 Massen-% Phospholipide;
- 0,5-5 Massen-% Polyoxylglyceride oder Makrogol-Glyceride, Polyoxyethylen-Fettsäureester oder Polyoxyethylen-Alkylether, vorzugsweise Polyoxyehtylen-Stearat (40).

20. Nanopartikel, wie sie in einem der Ansprüche 1 bis 10 definiert sind, für eine Verwendung als Träger für die Verabreichung eines interessierenden Moleküls.

21. Nanopartikel, wie sie in Anspruch 11 oder 12 definiert sind, für eine Verwendung als Medikament, vorzugsweise ein Medikament, das auf injizierbarem, oralem, kutanem oder nasalem Weg verabreicht wird.

## Claims

1. Anisotropic multicompartment lipid nanoparticles having a mean diameter of 10 to 500 nm, **characterized in that** they comprise a first lipophilic compartment only partly enclosed by a second hydrophilic compartment delimited by a phospholipid bilayer, said first compartment being only partly anchored to said second compartment,
and **characterized in that** said nanoparticles are stabilized by a mixture of surfactants comprising (a) phospholipids and (b) a mixture of nonionic surfactants comprising at least one three-segment derivative of the "hydrophobic-hydrophilic-hydrophobic" type having an HLB of greater than 11 according to the Griffin scale.

2. Nanoparticles according to Claim 1, where the polydispersity index is 5 to 15%.

3. Nanoparticles according to Claim 1 or 2, where the mean diameter is 100 to 250 nm.

4. Nanoparticles according to any one of Claims 1 to 3 where the lipophilic compartment consists of lipids in the liquid or semi-solid state at 25 °C.

5. Nanoparticles according to any one of Claims 1 to 4, where the lipophilic compartment comprises a fatty acid ester optionally mixed with a glyceride.

6. Nanoparticles according to Claim 5, where said fatty acid ester is selected from the group consisting of mixtures of polyethylene glycol mono- and diesters the molar mass of the hydrophilic part of which varies from 100 to 700 g/mol.

7. Nanoparticles according to Claim 5, where the glyceride is selected from the group consisting of mixtures of glycerol mono-, di- and triesters that are liquid or solid at 25 °C.

8. Nanoparticles according to any one of Claims 1 to 7, where the phospholipid bilayer comprises at least one phospholipid and one hydrophilic nonionic surfactant.

9. Nanoparticles according to Claim 8, where said phospholipid is selected from the group consisting of mixtures of zwitterionic phospholipids.

10. Nanoparticles according to Claim 8, where said hydrophilic nonionic surfactant is selected from the group consisting of polyoxylglycerides or macrogolglycerides, polyoxyethylene fatty acid esters and polyoxyethylene alkyl ethers

11. Nanoparticles according to any one of Claims 1 to 10, further comprising at least one molecule of interest.

12. Nanoparticles according to Claim 11, where the molecule of interest is selected from a nucleic acid, a protein, a polysaccharide and a molecule of 40 to 2000 Da.

13. Pharmaceutical composition comprising a suspension of nanoparticles as defined in any one of Claims 1 to 12 in a pharmaceutically acceptable excipient.

14. Method for preparing multicompartment lipid nanoparticles according to Claim 1 comprising the following steps:
- preparation of an oil/water emulsion comprising at least one lipid, phospholipids, a mixture of nonionic surfactants comprising at least one three-segment derivative of the "hydrophobic-hydrophilic-hydrophobic" type having an HLB of greater than 11 according to the Griffin scale, and optionally at least one molecule of interest;
- homogenization of said emulsion under high pressure.

15. Method according to Claim 14, further comprising a step of heating the emulsion to a temperature of 30 to 80 °C before its homogenization.

16. Method according to Claim 14, where said lipid is selected from the group consisting of mixtures of polyethylene glycol mono- and diesters the molar mass of the hydrophilic part of which varies from 100 to 700 g/mol.

17. Method according to Claim 14, where said phospholipid is selected from the group consisting of mixtures of zwitterionic phospholipids.

18. Method according to Claim 14, where said hydrophilic nonionic surfactant is selected from the group consisting of polyoxylglycerides or macrogolglycerides, polyoxyethylene fatty acid esters and polyoxyethylene alkyl ethers.

19. Method according to any one of Claims 14 to 18, where the oil/water emulsion comprises:
- 5-30% by mass lipids;
- 0.5-5% by mass phospholipids;
- 0.5-5% by mass polyoxylglycerides or macrogolglycerides, polyoxyethylene fatty acid esters or polyoxyethylene alkyl ethers, preferably polyoxyethylene (40) stearate.

20. Nanoparticles as defined in any one of Claims 1 to 10 for use as a carrier for administering a molecule of interest.

21. Nanoparticles as defined in Claim 11 or 12 for use as medicine, preferably a medicine administered by injection, orally, cutaneously or nasally.
